# DEMANDE DE BREVET EUROPEEN

(11) **EP 4 498 066 A1**
(43) Date de publication de la demande: **29.01.2025**
(21) Numéro de dépôt: 24190550.4
(22) Date de dépôt: 24.07.2024
(51) Int. Cl.: G01N 21/33, G01N 21/75, G01N 33/00, G01N 31/00

(54) **PROCÉDÉ D'ANALYSE CHIMIQUE EN PHASE GAZEUSE**

(30) Priorité: 27.07.2023 FR 2308127
(71) Demandeur: Hemera, 38240 Meylan (FR)
(72) Inventeur: MOUFLIH, Rachid, 38240 MEYLAN (FR)
(74) Mandataire: IP Trust

(57) **Abrégé**

Il est décrit un procédé d'analyse chimique d'un échantillon gazeux comprenant une première espèce, ladite première espèce présentant une activité spectroscopique, dite première activité, dans une gamme de longueurs d'ondes prédéterminée, le procédé d'analyse chimique comprenant :
a) une étape de conversion, dans une ampoule de conversion (20), de la première espèce en une deuxième espèce par réaction avec un réactif dit réactif de conversion, la deuxième espèce présentant une activité spectroscopique, dite deuxième activité, supérieure à la première activité ;
b) une étape d'acquisition d'un spectre d'absorption, par mesure spectroscopique, de l'échantillon gazeux après exécution de l'étape a) dans une cellule de mesure (30) reliée fluidiquement à l'ampoule de conversion (20) ;
c) une étape de traitement mathématique du spectre en vue d'en déduire la concentration de la première espèce dans l'échantillon gazeux.

## Description

### DOMAINE DE L'INVENTION

L'invention se rapporte au domaine des analyses chimiques et plus particulièrement des analyses chimiques en phase gazeuse.

Notamment, la présente invention concerne un procédé d'analyse chimique d'un échantillon gazeux comprenant au moins une espèce contaminante en vue de la détermination de la concentration de l'au moins une espèce contaminante dans ledit échantillon.

### ARRIERE PLAN TECHNOLOGIQUE DE L'INVENTION

La détection et la quantification des espèces contaminantes dans les gaz industriels est primordiale afin de garantir leur degré de pureté.

A cet égard, il est possible de mettre en oeuvre des techniques spectroscopiques qui sont non seulement stables dans le temps, et présentent l'avantage de pourvoir être mises en oeuvre relativement facilement. Néanmoins, il est des situations pour lesquelles les espèces contaminantes sont peu détectables et/identifiables par spectroscopie. C'est notamment le cas du H₂S. En effet, ce composé, dont l'activité spectroscopique est faible, est peu ou pas détectable dès lors que sa concentration dans un gaz est inférieure à 1 ppm.

Ainsi, un but de la présente invention est de proposer un procédé d'analyse chimique d'un échantillon gazeux comprenant une espèce contaminante et présentant une sensibilité supérieure aux procédés connus de l'homme du métier.

### BREVE DESCRIPTION DE L'INVENTION

L'invention concerne un procédé d'analyse chimique d'un échantillon gazeux comprenant une première espèce, ladite première espèce présentant une activité spectroscopique, dite première activité, dans une gamme de longueurs d'ondes prédéterminée, le procédé d'analyse chimique comprenant :
a) une étape de conversion, dans une cellule de conversion, de la première espèce en une deuxième espèce par réaction avec un réactif dit réactif de conversion, la deuxième espèce présentant une activité spectroscopique, dite deuxième activité, supérieure à la première activité ;
b) une étape d'acquisition d'un spectre d'absorption, par mesure spectroscopique, de l'échantillon gazeux après exécution de l'étape a) dans une cellule de mesure reliée fluidiquement à l'ampoule de conversion ;
c) une étape de traitement mathématique du spectre en vue d'en déduire la concentration de la première espèce dans l'échantillon gazeux.

Selon un mode de mise en oeuvre, la deuxième activité spectroscopique est au moins dix fois supérieure à la première activité spectroscopique.

Selon un mode de mise en oeuvre, la deuxième espèce présente un spectre d'absorption essentiellement périodique dans la gamme de longueurs d'ondes prédéterminées.

Selon un mode de mise en oeuvre, la première espèce comprend du H₂S, tandis que la deuxième espèce comprend du SO₂.

Selon un mode de mise en oeuvre, le réactif de conversion comprend du I₂O₅.

Selon un mode de mise en oeuvre, le réactif de conversion est sous l'une des formes choisis parmi : poudre, pastille, paillettes, granulés, billes.

Selon un mode de mise en oeuvre, l'étape b) comprend la mesure au moyen d'un spectrographe du spectre d'absorption par l'échantillon gazeux d'un rayonnement lumineux émis par une source lumineuse.

### BREVE DESCRIPTION DES DESSINS

D'autres caractéristiques et avantages de l'invention ressortiront de la description détaillée qui va suivre en référence à la figure annexée sur laquelle :
[Fig.1] La figure 1 est une représentation graphique d'un spectre d'absorption du H₂S entre 185 nm et 225 nm, l'axe vertical représentant le niveau d'absorption (en unité arbitraire) et l'axe vertical représentant la longueur d'onde en nm ;
[Fig.2] La figure 2 est une représentation graphique d'un spectre d'absorption du SO₂ entre 185 nm et 225 nm, l'axe vertical représentant le niveau d'absorption (en unité arbitraire) et l'axe vertical représentant la longueur d'onde en nm ;
[Fig.3] La figure 3 est une représentation schématique d'un dispositif d'analyse susceptible de mettre en oeuvre le procédé d'analyse selon la présente invention.

### DESCRIPTION DETAILLEE DE L'INVENTION

La présente invention concerne un procédé d'analyse chimique d'un échantillon gazeux comprenant un gaz porteur dans lequel est dilué une première espèce. Notamment la première espèce, selon les termes de la présente invention présente une activité spectroscopique, dite première activité, dans une gamme de longueurs d'ondes prédéterminée.

L'activité spectroscopique selon les termes de la présente invention peut se définir comme niveau d'absorption spectroscopique dans la gamme de longueurs d'ondes prédéterminée.

Ainsi, le procédé d'analyse selon la présente invention comprend une étape a) de conversion, dans une cellule de conversion, de la première espèce en une deuxième espèce par réaction avec un réactif dit réactif de conversion, la deuxième espèce présentant une activité spectroscopique, dite deuxième activité, supérieure à la première activité ;

Le procédé comprend également une étape b) d'acquisition d'un spectre d'absorption, par mesure spectroscopique, de l'échantillon gazeux après exécution de l'étape a) dans une cellule de mesure reliée fluidiquement à l'ampoule de conversion ;

L'étape b) est suivie d'une étape c) de traitement mathématique du spectre en vue d'en déduire la concentration de la première espèce dans l'échantillon gazeux.

Selon les termes de la présente invention, la première espèce présente une activité spectroscopique, dite première activité, dans la gamme de longueurs d'ondes prédéterminée, tandis que la deuxième espèce présente une activité spectroscopique, dite deuxième activité, dans la gamme de longueurs d'ondes prédéterminée. La deuxième activité spectroscopique peut par exemple être au moins 2 fois, avantageusement 10 fois, supérieure à la première activité spectroscopique.

En d'autres termes, et selon la présente invention, dans la gamme de longueurs d'ondes prédéterminées, le niveau maximal d'absorption (i.e. : activité spectroscopique) de la deuxième espèce est supérieur (avantageusement au moins deux fois, avantageusement 10 fois, supérieur) au niveau maximal d'absorption de la première espèce.

Par ailleurs, et de manière avantageuse, la deuxième espèce peut présenter, dans la gamme de longueurs d'ondes prédéterminée, une signature spécifique. Notamment, la deuxième espèce peut présenter un spectre d'absorption périodique ou pseudopériodique permettant une identification univoque de ladite deuxième espèce. Un spectre d'absorption périodique ou pseudopériodique prend notamment la forme d'un peigne.

A titre d'exemple, et sans que cela présente un caractère limitatif, il sera considéré dans la suite de l'énoncé que la première espèce est du H₂S tandis que la deuxième espèce est du SO₂. L'échantillon gazeux comprend donc un gaz porteur dans lequel est dilué du H₂S. Ce dernier peut être converti en SO₂ au moyen d'un réactif de conversion, par exemple du I₂O₅.

La réaction du conversion du H₂S en SO₂ par du I₂O₅ suit la réaction suivante :

5 H₂S+ 3 I₂O₅ → 3 I₂ + 5 SO₂ + 5 H₂O

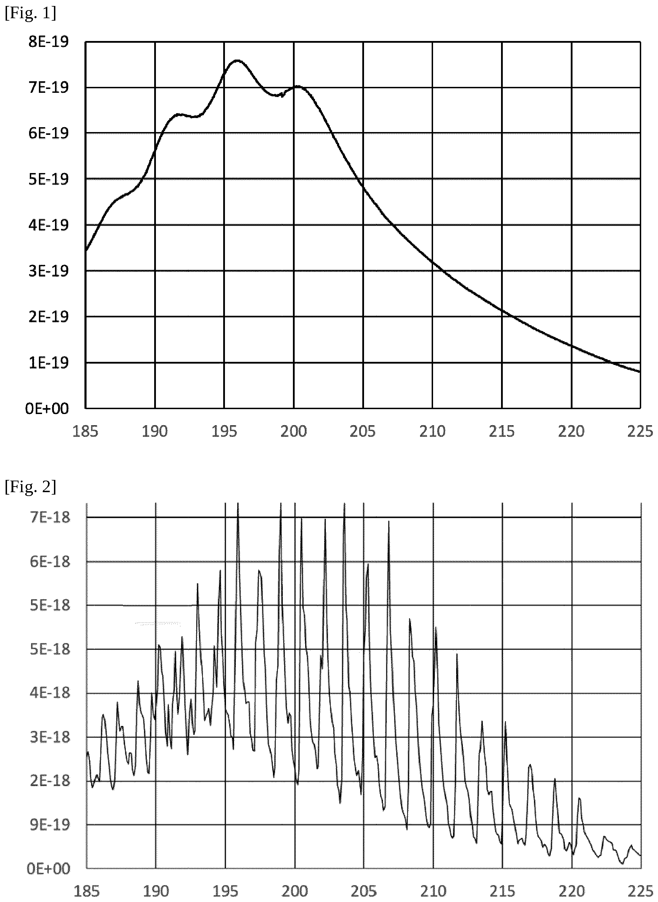

A titre d'exemple, la figure 1 est une représentation graphique du spectre d'absorption du H₂S (pris comme première espèce) dans la gamme de longueur d'ondes 185 nm - 225 nm, tandis que La figure 2 représente le spectre d'absorption du SO₂ (pris comme deuxième espèce) dans la même gamme de longueurs d'ondes.

Ces deux représentations graphiques permettent de constater que le niveau d'absorption du SO₂ dans cette gamme de longueurs d'onde est supérieur au niveau d'absorption du H₂S.

En d'autres termes, le seuil de détectabilité du H₂S par absorption spectroscopique est bien meilleur que celui du SO₂ . Ainsi, la conversion du H₂S en SO₂ permet de détecter la présence de H₂S à des concentrations moindres que par les techniques connue de l'état de la technique basées sur l'absorption directe du composé H₂S.

Ce dernier aspect permet d'améliorer la sensibilité de dosage et/ou d'analyse d'un composé chimique dilué dans un gaz porteur.

Par ailleurs, le caractère périodique du spectre d'absorption représenté à la figure 2 est caractéristique de la présence de SO₂. Plus particulièrement, la période mesurée atteste de la présence de SO₂, tandis l'amplitude des oscillations est associée à la concentration de SO₂ dans le gaz porteur.

Un traitement mathématique, impliquant notamment une transformée de Fourier, peut être mis en oeuvre pour la détermination de la concentration en SO₂ dans le gaz porteur.

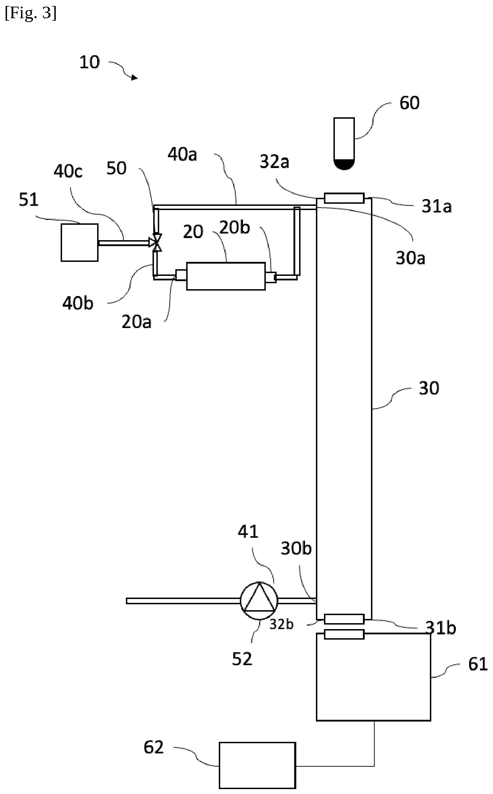

A titre d'exemple, le procédé d'analyse chimique selon la présente invention peut être mis en oeuvre par le dispositif d'analyse chimique 10 représenté à la figure 3.

Le dispositif d'analyse chimique 10 comprend notamment une ampoule de conversion 20 dans laquelle est disposée un réactif, dit de conversion, configuré pour convertir la première espèce de l'échantillon gazeux en la deuxième espèce.

L'ampoule de conversion 20 présente de manière générale une forme allongée, et peut, par exemple, être cylindrique. L'ampoule de conversion 20 comprend à l'une de ses extrémité une première entrée 20a et à l'autre de ses extrémités une première sortie 20b.

L'ampoule de conversion 20 comprend dans son volume un réactif, dit réactif de conversion, configuré pour convertir, par voie chimique, la première espèce susceptible de circuler sous forme gazeuse dans ladite ampoule en deuxième espèce.

Le réactif de conversion peut notamment comprendre du I₂O₅, par exemple sous forme de poudre, ou encore de cristaux. Aussi, afin de prévenir l'entraînement du I₂O₅ par le gaz porteur lors de son écoulement, il peut être considérer de mettre en oeuvre des membrane, par exemple des membrane en PTFE.

Le dispositif d'analyse chimique 10 comprend également des moyens spectroscopiques configurés pour mesurer un spectre absorption de l'échantillon gazeux après conversion du dichlore en dioxyde de chlore.

Plus particulièrement, les moyens spectroscopiques coopèrent avec une ampoule de mesure 30 à des fins de mesure du spectre d'absorption lorsque l'échantillon gazeux se trouve dans ladite ampoule de mesure.

Plus particulièrement, l'ampoule de mesure 30 de forme généralement allongée comprend deux extrémités 31a et 31b (opposées l'une de l'autre) pourvue chacune d'une fenêtre 32a et 32b transparente aux longueurs d'onde comprises dans le domaine d'analyse. Ces deux fenêtres 32a et 32b sont parallèles entre elles.

L'ampoule de conversion 20 peut comprendre un corps principal, de forme tubulaire et fermé hermétiquement à ses extrémités par des membranes de PTFE laissant passer les gaz et retenant le réactif de conversion.

Le corps principal peut de manière avantageuse être transparent et comprendre par exemple du PMMA ou un verre.

Les moyens spectroscopiques comprennent un spectrographe 61 et une source lumineuse 60 configurée pour émettre un rayonnement lumineux couvrant le domaine d'analyse.

Notamment, la source lumineuse 60 est disposée en regard de la fenêtre 32a afin de permettre une illumination d'un échantillon gazeux susceptible de se trouver dans l'ampoule de mesure 30.

Le spectrographe 61 est, pour sa part, disposé en regard de la fenêtre 32b et est agencé pour mesurer l'absorption du rayonnement lumineux par l'échantillon gazeux dans le domaine d'analyse. Il est entendu, sans qu'il soit nécessaire de le préciser qu'un spectrographe mesure un spectre d'absorption en fonction de la longueur d'onde. Le spectrographe 61 est avantageusement connecté à un calculateur 62 configuré pour traiter et/ou analyse les spectres collectés par ledit spectrographe 61.

Par « calculateur », on entend un dispositif électronique configuré pour exécuter un traitement automatique d'un spectre collecté par le spectrographe. Le calculateur 62 peut comprendre un espace mémoire, un processeur, une carte mère, un moyen d'affichage, une interface de contrôle.

Par ailleurs, l'ampoule de mesure 30 est fluidiquement connectée à l'ampoule de conversion 20. Notamment, un moyen de raccordement fluidique connecte la première sortie 20b à une entrée, dite deuxième entrée 30a disposée à proximité de l'extrémité 31a de l'ampoule de mesure 30. Le moyen de raccordement fluidique peut notamment comprendre un tube de raccordement fluidique, dit premier tube 40a. Le moyen de raccordement fluidique peut également être pourvu d'une vanne (ou une électrovanne) trois voies 50. Ainsi, le moyen de raccordement fluidique peut comprendre un tube 40b reliant la première sortie 20b à la vanne trois voies 50, le tube 40a reliant la vanne trois voies 50 à la deuxième entrée 30a, et enfin un tube 40c reliant une source de gaz porteur 51 à la vanne trois voies 50.

L'ampoule de mesure 30 comprend également une sortie, dite deuxième sortie 30b, disposée à proximité de l'extrémité 31b. Cette deuxième sortie 30b peut notamment être reliée à une pompe 52 au moyen d'une tube de raccordement fluidique, dit deuxième tube 41. Ce dernier agencement peut notamment être mis en oeuvre à des fins de purge et/ou de transfert de l'échantillon gazeux de l'ampoule de mesure.

La suite de l'énoncé est dévolue à la description du procédé d'analyse chimique d'un échantillon gazeux comprenant un gaz porteur dans lequel est dilué du H₂S. Notamment, le gaz porteur peut comprendre du diazote, du CH4, du H₂, sans toutefois limiter l'invention à ces seuls gaz.

Selon un mode de réalisation avantageux, le réactif de conversion comprend du I₂O₅, sous forme de poudre ou de monocristaux.

La réaction entre le H₂S et le I₂O₅ s'écrit comme suit :

5 H₂S+ 3 I₂O₅ → 3 I₂ + 5 SO₂ + 5 H₂O

Le procédé d'analyse chimique comprend également une étape b) d'acquisition d'un spectre d'absorption de l'échantillon gazeux après exécution de l'étape a) dans la cellule de mesure 30.

A cet égard, l'échantillon gazeux est transféré (étape a1)) de l'ampoule de conversion 20 vers la cellule de mesure 30 via les moyens de raccordement fluidique. Notamment, ce transfert peut impliquer un positionnement de la vanne trois voies 50 de manière à permettre la circulation de l'échantillon de gaz de la première sortie 20b vers la deuxième entrée 30a.

L'acquisition du spectre d'absorption comprend une illumination, par la source lumineuse 60, de l'échantillon gazeux présent dans la cellule de mesure 30 et une collecte du signal lumineux, après absorption par l'échantillon gazeux, par le spectrographe 61.

Dans la gamme de longueurs d'onde 185 nm - 225 nm, le SO₂ présente une signature spécifique en « peigne » tel qu'illustré à la figure 2.

L'invention comprend également une étape c) de traitement mathématique du spectre d'absorption en vue d'en déduire la concentration de SO₂ dans l'échantillon gazeux.

La connaissance de la concentration en SO₂ dans l'échantillon gazeux permet d'accéder à la concentration initiale en H₂S dans l'échantillon gazeux.

L'étape c) de traitement mathématique peut comprendre un calcul par transformée de Fourier du spectre d'absorption. Notamment, la transformé de Fourier du spectre de la figure 2 est une courbe en forme de cloche dont l'aire est représentative de la concentration en SO₂ présent dans l'échantillon gazeux. L'homme du métier reconnaîtra que des courbes d'étalonnage et/ou des abaques peuvent être mis en oeuvre pour l'interprétation des mesures effectuées.

De manière avantageuse, l'étape c) de traitement mathématique peut être exécutée au moyen du calculateur 62.

Ainsi, la présente invention propose de déterminer la concentration de H₂S diluée dans un gaz porteur en le convertissant en SO₂. Ce dernier présente, entre 185 nm et 225 nm, un niveau d'absorption supérieur à celui du H₂S, et permet ainsi de mesurer des concentrations en H₂S d'un échantillon en deçà des seuils usuellement admis lors de la mise en oeuvre des techniques d'analyse connues de l'homme du métier.

Le procédé d'analyse chimique peut comprendre une étape a0), précédent l'étape a), de mesure de fond qui comprend la mesure du spectre d'absorption d'un échantillon gazeux, dit de fond, formé uniquement du gaz porteur. Cette étape a0) comprend notamment l'acquisition d'un spectre d'absorption de gaz porteur (dépourvu de dichlore) présent dans la cellule de mesure selon des termes identiques à l'étape b).

Le gaz porteur peut notamment avoir été prélevé dans la source de gaz porteur 51.

Bien sûr, l'invention n'est pas limitée aux modes de réalisation décrits et on peut y apporter des variantes de réalisation sans sortir du cadre de l'invention tel que défini par les revendications.

## Revendications

1. Procédé d'analyse chimique d'un échantillon gazeux comprenant une première espèce, ladite première espèce présentant une activité spectroscopique, dite première activité, dans une gamme de longueurs d'ondes prédéterminée, le procédé d'analyse chimique comprenant :
a) une étape de conversion, dans une ampoule de conversion, de la première espèce en une deuxième espèce par réaction avec un réactif dit réactif de conversion, la deuxième espèce présentant une activité spectroscopique, dite deuxième activité, supérieure à la première activité ;
b) une étape d'acquisition d'un spectre d'absorption, par mesure spectroscopique, de l'échantillon gazeux après exécution de l'étape a) dans une cellule de mesure reliée fluidiquement à l'ampoule de conversion ;
c) une étape de traitement mathématique du spectre en vue d'en déduire la concentration de la première espèce dans l'échantillon gazeux.

2. Procédé d'analyse chimique selon la revendication 1, dans lequel la deuxième activité spectroscopique est au moins deux fois, avantageusement au moins dix fois, supérieure à la première activité spectroscopique.

3. Procédé d'analyse chimique selon la revendication 1 ou 2, dans lequel la deuxième espèce présente un spectre d'absorption essentiellement périodique dans la gamme de longueurs d'ondes prédéterminées.

4. Procédé d'analyse chimique selon l'une des revendications 1 à 3, dans lequel la première espèce comprend du H₂S, tandis que la deuxième espèce comprend du SO₂.

5. Procédé d'analyse chimique selon la revendication 4, dans lequel le réactif de conversion comprend du I₂O₅.

6. Procédé d'analyse chimique selon la revendication 5, dans lequel le réactif de conversion est sous l'une des formes choisis parmi : poudre, pastille, paillettes, granulés, billes.

7. Procédé d'analyse chimique selon l'une des revendications 1 à 6, dans lequel l'étape b) comprend la mesure au moyen d'un spectrographe du spectre d'absorption par l'échantillon gazeux d'un rayonnement lumineux émis par une source lumineuse.
